# EUROPÄISCHE PATENTSCHRIFT

(11) **EP 2 013 107 B1**
(45) Veröffentlichungstag und Bekanntmachung des Hinweises auf die Patenterteilung: **24.03.2010**
(21) Anmeldenummer: 07728443.8
(22) Anmeldetag: 24.04.2007
(51) Int. Cl.: B65D 79/02, G01N 33/46, G01N 31/22

(54) **PANEELVERPACKUNG MIT INDIKATOR**
WAINSCOT PANEL PACKAGE WITH INDICATOR
CONDITIONNEMENT DE LAMBRIS AVEC INDICATEUR

(30) Priorität: 02.05.2006 DE 102006020619
(43) Veröffentlichungstag der Anmeldung: 14.01.2009
(73) Patentinhaber: Fritz Egger GmbH & Co., 3105 Unterradlberg (AT)
(72) Erfinder: STEINWENDER, Martin, 2380 Perchtoldsdorf (AT)
(74) Vertreter: Cohausz & Florack
(86) Internationale Anmeldenummer: PCT/EP2007/053987
(87) Internationale Veröffentlichungsnummer: WO 2007/125058

(56) Entgegenhaltungen:
- EP-A1- 1 334 919
- WO-A-91/09726
- WO-A-02/071056
- WO-A-2004/101390
- DE-A1- 10 128 807
- DE-A1- 19 756 231
- DE-A1- 19 803 208
- DE-U1-202004 017 254
- GB-A- 2 258 918
- JP-A- 2005 329 983

## Beschreibung

Die Erfindung betrifft eine Paneelverpackung mit einer Umhüllung und mit mindestens einem von der Umhüllung aufgenommenen Paneel mit den Merkmalen des Oberbegriffs des Anspruchs 1 sowie ein Paneel mit den Merkmalen des Oberbegriffs des Anspruchs 16.

Paneelverpackungen sind seit langem bekannt. Dabei werden vielfältige Ausführungsformen in Bezug auf die Materialien, Größe, geometrische Form und optische Ausgestaltung der Umhüllungen gewählt. Insbesondere können Umhüllungen aus einem Kartonmaterial hergestellt sein, von denen ein oder mehrere Paneele aufgenommen werden.

Weiterhin ist es bekannt, Paneele mit Umhüllungen aus Verpackungsfolien zu verpacken. Auch eine Kombination eines Verpackungskartons sowie einer Verpackungsfolie ist aus dem Stand der Technik hinreichend bekannt.

Bei den bekannten Paneelverpackungen ist es von Nachteil, dass die Handhabung der Paneelverpackungen bzw. der Paneele durch eingeschränkte Informationen erschwert ist.

Zum einen ist dem Verleger bzw. Endverbraucher nur ein geringer Informationsgehalt über die Paneelverpackungen und/oder die Paneele ab Auslieferung zugänglich. Auch für den Hersteller weisen die bekannten Paneelverpackungen diese Nachteile auf, da er, sobald die Paneelverpackung seinen Einflußbereich verlassen hat, keine Möglichkeit besitzt, Informationen - abgesehen von der derzeitigen Lagerposition - der Paneelverpackung und/oder der Paneele zu erlangen. Insbesondere sind keine Paneelverpackungen bekannt, die Informationen individuell, also jeweils über sich selber zur Verfügung stellen können.

Zudem sind aus der WO 00/75417 und der WO 03/038777 Böden bekannt, bei denen Sensoren vorgesehen sind, die eine Beanspruchung des Bodens an eine Datenverarbeitungsanlage übermitteln können. Die Übermittlung kann dabei durch fest verbundene Leitungen oder über Funk realisiert sein. Solche Böden werden im verlegten Zustand zur Analyse von Beanspruchungen und zum Regeln bestimmter Zustände in Abhängigkeit der Beanspruchungen verwendet. Beispielsweise läßt sich so ein Boden realisieren, der beim Betreten einen Alarm auslöst oder eine Klimaanlage regelt. Bei den bekannten Böden ist es von Nachteil, dass die Sensoren aufwändig herzustellen sind und weitere Geräte zur Auswertung der Sensoren benötigt werden.

Einen weiteren Stand der Technik stellt die DE 101 28 807 A1 dar, welche die Merkmale des Oberbegriffs der Ansprüche 1 und 16 offenbart.

Der Erfindung liegt somit das technische Problem zugrunde, die Handhabung von Paneelverpackungen durch größeren Informationsumfang zu verbessern und zu erleichtern.

Das zuvor aufgezeigte technische Problem wird erfingdungsgemäß durch eine Paneelverpackung mit den Kennzeichnenden Merkmalen des Anspruchs 1 gelöst. Anspruch 16 hat ein entsprechend ausgestattetes Paneel zum Gegenstand. Der Indikator kann die Einhaltung der Verlegevorschriften anzeigen. Er ist erfindungsgemäß so gestaltet, dass an ihm abgelesen werden kann, ob die Paneelverpackung beispielsweise zwischen 15°C und 20°C, insbesondere bei mindestens 18°C und zwischen 30% und 80%, insbesondere zwischen 40% bis 70% Luftfeuchtigkeit über einen bestimmten Zeitraum von Tagen, insbesondere 24 oder 48 Stunden vorkonditioniert wurde.

Die Erfindung hat somit erkannt, dass durch Vorsehen eines Indikators die Möglichkeit gegeben wird, Informationen über eine Beanspruchung direkt, insbesondere mit dem bloßen Auge und ohne weitere Hilfsmittel, individuell an der Paneelverpackung abzulesen.

Als Indikatoren eignen sich beispielsweise alle Indikatoren, die in Salzen, die zur Trocknung Anwendung finden, verwendet werden, um zu signalisieren, ob die Salze bereits Wasser aufgenommen haben, oder ob sie noch zur Trocknung eingesetzt werden können.

Eine Änderung des Erscheinungsbildes kann durch eine Änderung der Farbe des Indikators ermöglicht werden. Weiterhin kann der Indikator verschiedene Felder aufweisen, die beispielsweise zwei verschiedene Zustände annehmen können. So kann eine Änderung des Erscheinungsbildes eine Änderung der Anzahl der Felder eines gleichen Zustandes sein.

Dadurch, dass der Indikator insbesondere so ausgestaltet sein kann, dass er nur einmal sein Erscheinungsbild ändert, ist eine Paneelverpackung mit einem Indikator angegeben, die eine einmal eingetretene Beanspruchung archivieren kann. Diese Ausführungsform eignet sich besonders zur Aufklärung von Schadensfällen, da der Indikator als Nachweis eines Über- bzw. Unterschreitens vorgegebener Grenzwerte dienen kann.

Das oben aufgezeigte technische Problem wird unabhängig von der Verpackung der Paneele auch durch ein Paneel mit einem zuvor genannten Indikator gemäß Anspruch 16 gelöst. Der Indikator wird im Folgenden näher beschrieben, wobei sich das Beschriebene unabhängig auf eine Paneelverpackung mit Indikator oder auf ein Paneel mit einem Indikator anwenden lässt.

Weiterhin kann vorgesehen sein, dass der Indikator mehrfach, insbesondere ständig in Abhängigkeit von der mindestens einen Beanspruchung sein Erscheinungsbild ändert. So kann jederzeit festgestellt werden, in welchem Zustand sich die Paneelverpackung bzw. die Paneele zu einem bestimmten Zeitpunkt befinden. In Abhängigkeit von diesem Zustand können dann Entscheidungen bezüglich der Verlegbarkeit der Paneele getroffen werden und die Handhabung der Paneelverpackung bzw. der Paneele erheblich vereinfacht werden.

Dabei kann die mindestens eine Beanspruchung eine durch Temperatur und/oder Feuchtigkeit bedingte Beanspruchung sein. So kann ein Unter- bzw. Überschreiten von klimatischen Grenzwerten bezüglich der zulässigen Temperatur und/oder Feuchtigkeit während des Transports sichtbar gemacht werden. Insbesondere kann dies bei Containertransporten über große Distanzen von großem Vorteil sein.

Weiterhin kann ein Indikator vorgesehen sein, der eine durch mechanische Spannung bedingte Beanspruchung anzeigen kann. Beispielsweise kann sich ein solcher Indikator bei einer zu großen Belastung der Paneelverpackung verfärben. Auch kann ein solcher Indikator als verkapseltes System ausgeführt sein und an einem oder allen Paneelen vorgesehen sein. Bei einer zu großen Belastung setzen die Kapseln einen Farbstoff frei. So können kleinste Beschädigungen der Paneele, beispielsweise der Kantenbereiche, sichtbar gemacht werden und der Verleger kann die entsprechenden Paneele zum Verlegen in den Randbereichen sowie durch Abtrennen der beschädigten Kanten verwenden.

Alternativ kann die mindestens eine Beanspruchung eine durch Licht bedingte Beanspruchung sein. So kann an dem Indikator abgelesen werden, ob die Paneelverpackung und insbesondere ein Paneel einer zu starken Belastung durch Licht ausgesetzt wurde. Dadurch kann verhindert werden, dass ein etwaiges Verblassen des Dekors eines Paneels erst nach dem Verlegen festgestellt wird.

Besonders vorteilhaft kann der Indikator an mindestens einem Befestigungselement angebracht sein.

Dabei kann das mindestens eine Befestigungselement eine Verpackungsfolie der Umhüllung sein. Wenn der Indikator von außen auf der Paneelverpackung aufgebracht ist, kann er zur Anzeige der Belastungen, die von außen auf die Paneelverpackung wirken, verwendet werden. So kann ein Indikator auch an der Außenseite des Bodens der Paneelverpackung vorgesehen sein. Wird die Paneelverpackung dann eine bestimmte Zeit auf dem Boden, auf dem die Paneele verlegt werden sollen, belassen, kann im Anschluss anhand des Indikators abgelesen werden, ob der Boden die nötige Trockenheit aufweist.

Wird der Indikator, oder ein weiterer Indikator an der Innenseite der Verpackungsfolie angebracht, so kann die Beanspruchung nach dem Öffnen der Paneelverpackung angezeigt werden. Es können auch mehrere Indikatoren vorgesehen sein, beispielsweise auf der Außen- und Innenseite der Verpackungsfolie. So kann eine Konditionierung der verschlossenen Paneelverpackung in dem Raum, in dem die Paneele verlegt werden sollen, an den angebrachten Indikatoren abgelesen werden. Es kann dann vorgeschrieben sein, dass ein Öffnen der Paneelverpackung und anschließendes Verlegen erst nach einem Angleichen der Indikatoren zulässig ist.

Ebenso kann das mindestens eine Befestigungselement ein Verpackungskarton sein. Besonders bei Verwendung einer Paneelverpackung, die aus einer Verpackungsfolie und einem Verpackungskarton besteht ist dies vorteilhaft, da so nach Entfernen der Verpackungsfolie die Paneele zum Konditionieren in dem Verpackungskarton verbleiben können, wobei direkt an dem Verpackungskarton der Zustand der Paneele durch den Indikator angezeigt werden kann.

Weiterhin kann das mindestens eine Befestigungselement ein Paneel sein. So kann beispielsweise an jedem Paneel individuell angezeigt werden, welchen Beanspruchungen es ausgesetzt war oder ist. Insbesondere kann so ein Verlegen eines Paneels im falsch konditionierten Zustand und/oder auf einem zu feuchten Untergrund angezeigt werden. Auch kann ein unzulässiger Fremdfeuchteangriff, beispielsweise durch Verwendung von zu viel Reinigungsflüssigkeit, durch diesen Indikator angezeigt werden.

Eine kostengünstige Möglichkeit ist es, wenn das mindestens eine Befestigungselement ein Einleger bzw. ein Etikett ist. Dann können die Indikatoren in großen Stückzahlen hergestellt werden und den Paneelverpackungen beigelegt werden bzw. an den Paneelverpackungen und/oder Paneelen befestigt werden.

Falls ein Paneel ein Trittschallmaterial aufweist, kann dieses ebenfalls als Befestigungselement dienen.

Eine Ausführungsform ist dadurch gekennzeichnet, dass der Indikator eine Abdeckung aufweist. Insbesondere kann es sich dabei um eine den Indikator deaktivierende Abdeckung handeln. Erst nachdem die Abdeckung entfernt worden ist passt sich der Indikator an die Umgebung an. So könnte ein Indikator an der Innenseite der Verpackungsfolie vorgesehen sein, der von einem zusätzlichen Verpackungskarton der Paneele oder durch ein Paneel selbst deaktiviert ist. Der Indikator kann dann so "programmiert" sein, dass sich beim Öffnen der Paneelverpackung und somit dem Aktivieren des Indikators durch Entfernen der Abdeckung ab einem bestimmten Temperatur- und/oder Feuchteunterschied zwischen dem Indikator und der Umgebungsluft der Indikator irreversibel verfärbt und so als Nachweis einer unzureichenden Konditionierung der Paneele dienen kann. Umgekehrt kann der Indikator an dem untersten Paneel in der Paneelverpackung angeordnet sein und die Abdeckung mit der Paneelverpackung, beispielsweise mit einer Verpackungsfolie oder einem Verpackungskarton verbunden sein, so dass die Abdeckung beim Entnehmen des letzten Paneels den Indikator freigibt und wie oben erläutert entsprechend Beanspruchungen anzeigen kann.

Besonders bevorzugt ist es, wenn mindestens eine das Erscheinungsbild des Indikators erläuternde Skala vorgesehen ist. Eine Skala kann dabei unmittelbar neben dem Indikator vorgesehen sein. Beispielsweise kann eine Skala auf der Außenseite und/oder Innenseite der Verpackungsfolie vorgesehen sein. Es kann sich um eine Vergleichsskala handeln, die bestimmte Farben und deren Bedeutung in Bezug auf die Temperatur und/oder Feuchtigkeit des Indikators erläutert. Die Skala kann aufgedruckt werden.

Es ist auch möglich, einen Indikator auf das Befestigungselement aufzudrucken. So kann durch eine flexible Produktion je nach Bedarf eine Paneelverpackung mit einem Indikator versehen werden.

Es können gleiche Indikatoren mit gleichen oder unterschiedlichen Skalen verwendet werden. Beispielsweise kann der gleiche Indikator am Boden und an der Seite der Paneelverpackung vorgesehen sein. Am Boden könnte eine Skala "Bodenkondition", an der Seite eine Skala "Umgebungskondition" angebracht sein. Auch können je nach Funktion unterschiedliche Indikatoren vorgesehen sein Insbesondere kann die Zusammensetzung des Indikators an den jeweils anzuzeigenden Zustand angepasst sein.

Die für einen Verleger bzw. bei der Verlegung durch insbesondere ständiges Ändern des Erscheinungsbildes des Indikators beispielsweise anzeigbaren Beanspruchungen können dabei mittelbar zur Anzeige von weiteren Zuständen der Paneele dienen.

Durch entsprechende Ausgestaltung kann ein Indikator, der eine Beanspruchung oder eine Kombination von Beanspruchungen durch Temperatur und/oder Feuchte bzw. mechanischer Beanspruchung anzeigt dazu verwendet werden, eine Aussage über das Risiko von Peaking (Kantenhochzug), Verzug, Verwerfungen, Längendehnungen, Bananenproblem, Winkelproblem oder sonstiger Probleme, die durch nicht ausreichende Konditionierung der in der Paneelverpackung befindlichen Paneele entstehen können, zu treffen. Je nach Anforderung kann der Indikator, wie bereits erwähnt, dabei an der Paneelverpackung und/oder den Paneelen vorgesehen sein.

Die für den Hersteller bzw. die Produktion durch insbesondere einmaliges Ändern des Erscheinungsbildes des Indikators beispielsweise anzeigbaren Beanspruchungen können dabei ebenfalls mittelbar zur Anzeige von weiteren Zuständen der Paneele dienen. Besonders vorteilhaft kann so der Nachweis für eine etwaige Reklamationsbearbeitung erfolgen.

Bei den mittelbar festzustellenden Beanspruchungen seien, jedoch nicht abschließend, gebannt: zu feuchter Untergrund, zu feuchter Transport, zu heiße Vorlauftemperatur einer Bodenheizung, zu feuchtes/trockenes Umgebungsklima, zu feuchte Reinigung oder Wasserschäden sowie mechanische Beschädigungen durch den Endverbraucher.

Die vorliegende Erfindung soll im Folgenden anhand spezieller Ausführungsbeispiele sowie der beigefügten Zeichnung näher erläutert werden. Die Zeichnung zeigt in
- Fig. 1: eine Paneelverpackung mit einem an der Verpackungsfolie befestigten Indikator,
- Fig. 2: eine geöffnete Paneelverpackung mit einem an der Verpackungsfolie und einem an dem Verpackungskarton befestigten Indikator, jeweils mit einer erläuternden Skala und in
- Fig. 3: eine Paneelverpackung mit einem an dem Verpackungskarton befestigten Indikator sowie einem an einem Paneel befestigten Indikator.

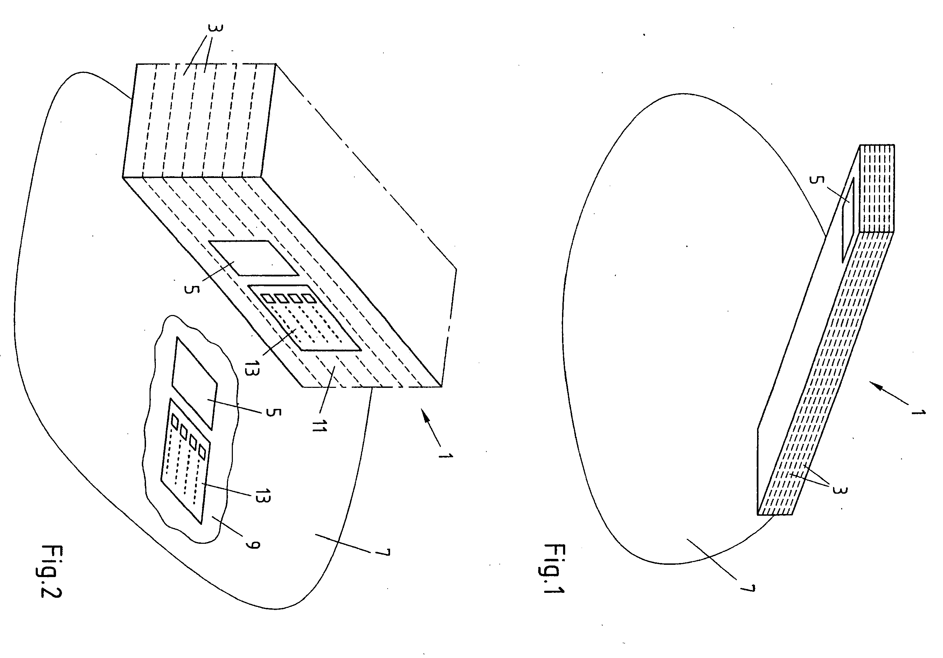

Fig. 1 zeigt eine Paneelverpackung 1 mit mehreren von der Paneelverpackung 1 aufgenommenen Paneelen 3 und mit einem Indikator 5 zur Anzeige von einer Beanspruchung durch Temperatur und Feuchtigkeit, wobei der Indikator 5 einmal in Abhängigkeit von der Beanspruchung sein Erscheinungsbild ändert. Dabei kann die Umhüllung der Paneelverpackung aus einer Verpackungsfolie bestehen.

Eine solche Ausführungsform ist besonders geeignet um ein etwaiges Unter- oder Überschreiten zulässiger Grenzwerte anzuzeigen. Sollte sich die Feuchtigkeit beispielsweise während des Transports nicht zwischen 30% und 80%, insbesondere zwischen 40% bis 70% befunden haben, könnte sich der Indikator 5 dauerhaft rot verfärben und somit die Unbrauchbarkeit der Paneele 3 signalisieren und gleichzeitig Reklamationen ausschließen, da die Transportvorschriften nicht eingehalten wurden.

Alternativ kann der Indikator 5 für eine Auflage auf einem Boden 7 vorgesehen sein. Durch Belassen der Paneelverpackung 1 auf dem Boden 7 für einen vorgeschriebenen Zeitraum kann der Indikator 5 dazu verwendet werden, eine Aussage über die Trockenheit des Bodens 7 zu treffen.

Fig. 2 zeigt eine geöffnete Paneelverpackung 1, mit Paneelen 3. Die Umhüllung wird in dem in Fig. 2 gezeigten Ausführungsbeispiel aus einer Verpackungsfolie 9 und einem Verpackungskarton 11 gebildet. Vor dem Öffnen der Verpackungsfolie 9 war der Verpackungskarton 11 von der Verpackungsfolie 9 umschlossen. Die Verpackungsfolie 9 sowie der Verpackungskarton 11 weisen jeweils Indikatoren 5 und eine jeweils einen Indikator 5 erläuternde Skala 13 auf.

Die Skala 13 kann beispielsweise vier Felder verschiedener Farbe aufweisen. Zusätzlich ist neben jedem Feld ein die Farbe erläuternder Text vorgesehen.

Die in Fig. 2 gezeigte Ausführungsform kann dazu verwendet werden, dass zunächst die Paneelverpackung 1 im geschlossenen Zustand über einen bestimmten Zeitraum von Tagen, insbesondere 24 oder 48 Stunden vorkonditioniert wird. Dabei nimmt der an der Verpackungsfolie 9 angebrachte Indikator 5 - der an der im verpackten Zustand nach außen gerichteten Fläche der Verpackungsfolie 9 angebracht ist - eine bestimmte Farbe an, die auf der Skala 13 als verlegetauglich erläutert wird.

Es kann dann vorgeschrieben sein, dass ein Öffnen der Paneelverpackung 1 und ein Verlegen der Paneele 3 nicht vor dem Angleichen der beiden Indikatoren 5 erfolgen darf. Somit kann sichergestellt werden, dass sich die Paneele 3 ausreichend lange an die Umgebungsbedingungen anpassen konnten.

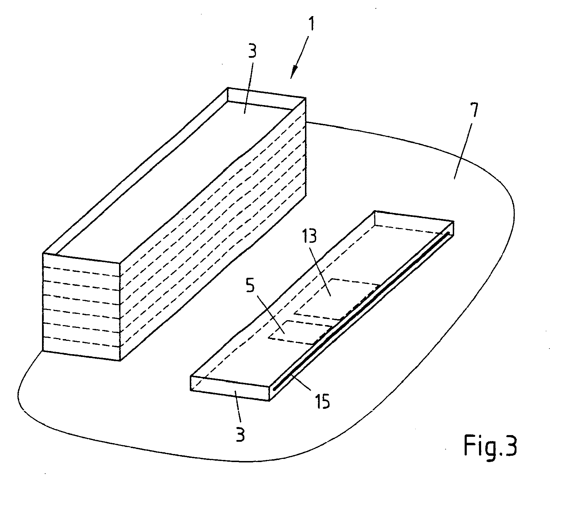

Fig. 3 zeigt eine geöffnete Paneelverpackung 1, aus der ein Paneel 3 entnommen wurde. Das Paneel 3 weist an seiner Unterseite einen Indikator 5 auf. Insbesondere kann der Indikator 5 an einer Trittschalldämmung befestigt sein. Zusätzlich ist an den Längskanten des Paneels 3 ein Indikator 15 vorgesehen. Der Indikator 5 kann, wie zuvor erläutert, zur Anzeige der Feuchtigkeit des Bodens 7 verwendet werden. Ein Teil der Skala 13, die in Fig. 3 ebenfalls an der Unterseite des Paneels 3 vorgesehen ist, dient zur Erläuterung des Indikators 5.

Der Indikator 15 kann verkapselt ausgeführt sein, so dass er bei zu großer mechanischer Beanspruchung der Längskanten eine auf der Skala 13 erläuterte Verfärbung aufweist.

Ebenso kann der Indikator 15 derart ausgeführt sein, dass er ein Peaking der Längskante oder einen Verzug des Paneels 3 durch Änderung seines Erscheinungsbildes anzeigen kann.

Die Erfindung ist nicht auf die zuvor genannten Ausführungsbeispiele beschränkt. Insbesondere kann der Indikator mit einem Einleger oder Etikett verbunden sein. Der Indikator kann durch Drucken an der Paneelverpackung und/oder einem oder mehreren Paneelen vorgesehen werden.

Zudem kann eine Abdeckung für den Indikator vorgesehen sein.

## Patentansprüche

1. Paneelverpackung,
- mit einer Umhüllung,
- mit mindestens einem von der Umhüllung aufgenommenen Paneel (3) und
- mit mindestens einem Indikator (5,15) zur Anzeige von mindestens einer Beanspruchung,
- wobei der Indikator (5,15) an mindestens einem Befestigungselement angebracht ist und
- wobei der Indikator (5,15) mindestens einmal in Abhängigkeit von der mindestens einen Beanspruchung sein Erscheinungsbild ändert,
**dadurch gekennzeichnet,**
- **dass** mindestens ein Indikator so gestaltet ist, dass die Einhaltung einer Vorkonditionierung bei einer Temperatur zwischen 15°C und 20°C und einer Luftfeuchtigkeit zwischen 30% und 80% über einen bestimmten Zeitraum von Tagen, insbesondere 24 oder 48 Stunden, ablesbar ist.

2. Paneelverpackung nach Anspruch 1,
**dadurch gekennzeichnet,**
**dass** der Indikator (5,15) mehrfach, insbesondere ständig in Abhängigkeit von der mindestens einen Beanspruchung sein Erscheinungsbild ändert.

3. Paneelverpackung nach Anspruch 1 oder 2,
**dadurch gekennzeichnet,**
**dass** die mindestens eine Beanspruchung eine durch Temperatur bedingte Beanspruchung ist.

4. Paneelverpackung nach einem der Ansprüche 1 bis 3,
**dadurch gekennzeichnet,**
**dass** die mindestens eine Beanspruchung eine durch Feuchtigkeit bedingte Beanspruchung ist.

5. Paneelverpackung nach einem der Ansprüche 1 bis 4,
**dadurch gekennzeichnet,**
**dass** die mindestens eine Beanspruchung eine durch mechanische Spannung bedingte Beanspruchung ist.

6. Paneelverpackung nach einem der Ansprüche 1 bis 5,
**dadurch gekennzeichnet,**
**dass** die mindestens eine Beanspruchung eine durch Licht bedingte Beanspruchung ist.

7. Paneelverpackung nach einem der Ansprüche 1 bis 6,
**dadurch gekennzeichnet,**
**dass** das mindestens eine Befestigungselement eine Verpackungsfolie (9) ist.

8. Paneelverpackung nach einem der Ansprüche 1 bis 7,
**dadurch gekennzeichnet,**
**dass** das mindestens eine Befestigungselement ein Verpackungskarton (11) ist.

9. Paneelverpackung nach einem der Ansprüche 1 bis 8,
**dadurch gekennzeichnet,**
**dass** das mindestens eine Befestigungselement ein Paneel (3) ist.

10. Paneelverpackung nach einem der Ansprüche 1 bis 9,
**dadurch gekennzeichnet,**
**dass** das mindestens eine Befestigungselement ein Einleger ist.

11. Paneelverpackung nach einem der Ansprüche 1 bis 10,
**dadurch gekennzeichnet,**
**dass** das mindestens eine Befestigungselement ein Etikett ist.

12. Paneelverpackung nach einem der Ansprüche 1 bis 11,
**dadurch gekennzeichnet,**
**dass** das mindestens eine Befestigungselement ein Trittschallmaterial ist.

13. Paneelverpackung nach einem der Ansprüche 1 bis 12,
**dadurch gekennzeichnet,**
**dass** der Indikator (5,15) eine Abdeckung aufweist.

14. Paneelverpackung nach einem der Ansprüche 1 bis 13,
**dadurch gekennzeichnet,**
**dass** mindestens eine das Erscheinungsbild des Indikators (5,15) erläuternde Skala (13) vorgesehen ist.

15. Paneelverpackung nach einem der Ansprüche 1 bis 14,
**dadurch gekennzeichnet,**
**dass** der Indikator (5,15) auf das Befestigungselement aufgedruckt ist.

16. Paneel,
- mit mindestens einem Indikator (5,15) zur Anzeige von mindestens einer Beanspruchung,
- wobei der Indikator (5,15) an mindestens einem Befestigungselement angebracht ist und
- wobei der Indikator (5,15) mindestens einmal in Abhängigkeit von der mindestens einen Beanspruchung sein Erscheinungsbild ändert,
**dadurch gekennzeichnet,**
- **dass** mindestens ein Indikator so gestaltet ist, dass die Einhaltung einer Vorkonditionierung bei einer Temperatur zwischen 15°C und 20°C und einer Luftfeuchtigkeit zwischen 30% und 80% über einen bestimmten Zeitraum von Tagen, insbesondere 24 oder 48 Stunden, ablesbar ist.

17. Paneel nach Anspruch 16,
**dadurch gekennzeichnet,**
**dass** der Indikator (5,15) mehrfach, insbesondere ständig in Abhängigkeit von der mindestens einen Beanspruchung sein Erscheinungsbild ändert.

18. Paneel nach Anspruch 16 oder 17,
**dadurch gekennzeichnet,**
**dass** die mindestens eine Beanspruchung eine durch Temperatur bedingte Beanspruchung ist.

19. Paneel nach einem der Ansprüche 16 bis 18,
**dadurch gekennzeichnet,**
**dass** die mindestens eine Beanspruchung eine durch Feuchtigkeit bedingte Beanspruchung ist.

20. Paneel nach einem der Ansprüche 16 bis 19,
**dadurch gekennzeichnet,**
**dass** die mindestens eine Beanspruchung eine durch mechanische Spannung bedingte Beanspruchung ist.

21. Paneel nach einem der Ansprüche 16 bis 20,
**dadurch gekennzeichnet,**
**dass** die mindestens eine Beanspruchung eine durch Licht bedingte Beanspruchung ist.

22. Paneel nach einem der Ansprüche 16 bis 21,
**dadurch gekennzeichnet,**
**dass** das mindestens eine Befestigungselement ein Paneel (3) ist.

23. Paneel nach einem der Ansprüche 16 bis 22,
**dadurch gekennzeichnet,**
**dass** das mindestens eine Befestigungselement ein Etikett ist.

24. Paneel nach einem der Ansprüche 16 bis 23,
**dadurch gekennzeichnet,**
**dass** das mindestens eine Befestigungselement ein Trittschallmaterial ist.

25. Paneel nach einem der Ansprüche 16 bis 24,
**dadurch gekennzeichnet,**
**dass** der Indikator (5,15) eine Abdeckung aufweist.

26. Paneel nach einem der Ansprüche 16 bis 25,
**dadurch gekennzeichnet,**
**dass** mindestens eine das Erscheinungsbild des Indikators (5,15) erläuternde Skala (13) vorgesehen ist.

27. Paneel nach einem der Ansprüche 16 bis 26,
**dadurch gekennzeichnet,**
**dass** der Indikator (5,15) auf das Befestigungselement aufgedruckt ist.

## Claims

1. A panel package,
- comprising a wrapping,
- comprising at least one panel (3) accommodated by the wrapping and
- comprising at least one indicator (5, 15) for displaying at least one stress,
- wherein the indicator (5, 15) is fixed to at least one fastening element and
- wherein the indicator (5, 15) changes its appearance at least once as a function of the at least one stress,
**characterized in**
- **that** at least one indicator is designed in such a manner that the compliance with a preconditioning at a temperature of between 15°C and 20°C and a humidity of between 30% and 80% over a certain period of days, in particular 24 or 48 hours, can be read.

2. The panel package according to claim 1,
**characterized in**
**that** the indicator (5, 15) changes its appearance repeatedly, in particular constantly, as a function of the at least one stress.

3. The panel package according to claim 1 or 2,
**characterized in**
**that** the at least one stress is a stress caused by temperature.

4. The panel package according to one of claims 1 to 3,
**characterized in**
**that** the at least one stress is a stress caused by moisture.

5. The panel package according to one of claims 1 to 4,
**characterized in**
**that** the at least one stress is a stress caused by mechanical stress.

6. The panel package according to one of claims 1 to 5,
**characterized in**
**that** the at least one stress is a stress caused by light.

7. The panel package according to one of claims 1 to 6,
**characterized in**
**that** the at least one fastening element is a packaging film (9).

8. The panel package according to one of claims 1 to 7,
**characterized in**
**that** the at least one fastening element is a packaging board (11).

9. The panel package according to one of claims 1 to 8,
**characterized in**
**that** the at least one fastening element is a panel (3).

10. The panel package according to one of claims 1 to 9,
**characterized in**
**that that** the at least one fastening element is an insert.

11. The panel package according to one of claims 1 to 10,
**characterized in**
**that** the at least one fastening element is a label.

12. The panel package according to one of claims 1 to 11,
**characterized in**
**that** the at least one fastening element is an impact sound insulation material.

13. The panel package according to one of claims 1 to 12,
**characterized in**
**that** the indicator (5, 15) encompasses a cover.

14. The panel package according to one of claims 1 to 13,
**characterized in**
**that** provision is made for at least one scale (13), which explains the appearance of the indicator (5, 15).

15. The panel package according to one of claims 1 to 14,
**characterized in**
**that** the indicator (5, 15) is imprinted onto the fastening element.

16. A panel
- comprising at least one indicator (5, 15) for displaying at least one stress,
- wherein the indicator (5, 15) is fixed to at least one fastening element and
- wherein the indicator (5, 15) changes its appearance at least once as a function of the at least one stress,
**characterized in**
- **that** at least one indicator is designed in such a manner that the compliance with a preconditioning at a temperature of between 15°C and 20°C and a humidity of between 30% and 80% over a certain period of days, in particular 24 or 48 hours, can be read.

17. The panel according to claim 16,
**characterized in**
**that** the indicator (5, 15) changes its appearance repeatedly, in particular constantly, as a function of the at least one stress.

18. The panel according to claim 16 or 17,
**characterized in**
**that** the at least one stress is a stress caused by temperature.

19. The panel according to one of claims 16 to 18,
**characterized in**
**that** the at least one stress is a stress caused by moisture.

20. The panel according to one of claims 16 to 19,
**characterized in**
**that** the at least one stress is a stress caused by mechanical stress.

21. The package according to one of claims 16 to 20,
**characterized in**
**that** the at least one stress is a stress caused by light.

22. The panel according to one of claims 16 to 21,
**characterized in**
**that** the at least one fastening element is a panel (3).

23. The panel according to one of claims 16 to 22,
**characterized in**
**that** the at least one fastening element is a label.

24. The panel according to one of claims 16 to 23,
**characterized in**
**that** the at least one fastening element is an impact sound insulation material.

25. The panel according to one of claims 16 to 24,
**characterized in**
**that** the indicator (5, 15) encompasses a cover.

26. The panel according to one of claims 16 to 25,
**characterized in**
**that** provision is made for at least one scale(13), which explains the appearance of the indicator (5, 15).

27. The panel according to one of claims 16 to 26,
**characterized in**
**that** the indicator (5, 15) is imprinted onto the fastening element.

## Revendications

1. Conditionnement de lambris,
- avec une enveloppe,
- avec au moins un lambris (3), qui est reçu par l'enveloppe, et
- avec, au moins, un indicateur (5, 15) pour la signalisation d'au moins une contrainte,
- l'indicateur (5, 15) étant installé sur au moins un élément de fixation, et
- l'indicateur (5, 15) modifiant au moins une fois son apparence en fonction de la contrainte au moins exercée,
**caractérisé en ce que**
- un indicateur au moins est conçu de sorte qu'il soit possible de lire si le pré-conditionnement à une température située entre 15 °C et 20 °C et une humidité de l'air située entre 30 % et 80 %, pendant une période d'un certain nombre de jours, en particulier de 24 ou 48 heures, est respecté.

2. Conditionnement de lambris selon la revendication 1, **caractérisé en ce que** l'indicateur (5, 15) modifie son apparence, plusieurs fois, en particulier continuellement, en fonction de la contrainte au moins exercée.

3. Conditionnement de lambris selon revendication 1 ou 2, **caractérisé en ce que** la contrainte au moins exercée est une contrainte thermique.

4. Conditionnement de lambris selon l'une des revendications 1 à 3, **caractérisé en ce que** la contrainte au moins exercée est une contrainte qui est due à l'humidité.

5. Conditionnement de lambris selon l'une des revendications 1 à 4, **caractérisé en ce que** la contrainte au moins exercée est une contrainte mécanique.

6. Conditionnement de lambris selon l'une des revendications 1 à 5, **caractérisé en ce que** la contrainte au moins exercée est une contrainte qui est due à la lumière.

7. Conditionnement de lambris selon l'une des revendications 1 à 6, **caractérisé en ce que** l'élément de fixation au moins prévu est une feuille de conditionnement (9).

8. Conditionnement de lambris selon l'une des revendications 1 à 7, **caractérisé en ce que** l'élément de fixation au moins prévu est un carton de conditionnement (11).

9. Conditionnement de lambris selon l'une des revendications 1 à 8, **caractérisé en ce que** l'élément de fixation au moins prévu est un lambris (3).

10. Conditionnement de lambris selon l'une des revendications 1 à 9, **caractérisé en ce que** l'élément de fixation au moins prévu est un insert.

11. Conditionnement de lambris selon l'une des revendications 1 à 10, **caractérisé en ce que** l'élément de fixation au moins prévu est une étiquette.

12. Conditionnement de lambris selon l'une des revendications 1 à 11, **caractérisé en ce que** l'élément de fixation au moins prévu est un matériau atténuant les bruits de pas.

13. Conditionnement de lambris selon l'une des revendications 1 à 12, **caractérisé en ce que** l'indicateur (5, 15) présente un recouvrement.

14. Conditionnement de lambris selon l'une des revendications 1 à 13, **caractérisé en ce qu'**est prévue une échelle (13), qui explique l'apparence de l'indicateur (5, 15).

15. Conditionnement de lambris selon l'une des revendications 1 à 14, **caractérisé en ce que** l'indicateur (5, 15) est imprimé sur l'élément de fixation.

16. Lambris,
- avec au moins un indicateur (5, 15) pour l'indication d'au moins une contrainte,
- l'indicateur (5, 15) étant installé sur au moins un élément de fixation, et
- l'indicateur (5, 15) modifiant an moins une fois son apparence en fonction de la contrainte au moins exercée,
**caractérisé en ce que**
- au moins un indicateur est conçu de sorte qu'il soit possible de lire si un pré-conditionnement à une température située entre 15 °C et 20 °C et une humidité de l'air située entre 30 % et 80 %, pendant une période d' un certain nombre de jours, en particulier de 24 ou de 48 heures, est respecté.

17. Lambris selon la revendication 16, **caractérisé en ce que** l'indicateur (5, 15) modifie son apparence plusieurs fois, en particulièrement continuellement, en fonction de la contrainte au moins exercée.

18. Lambris selon revendication 16 ou 17, **caractérisé en ce que** la contrainte au moins exercée est une contrainte thermique.

19. Lambris selon l'une des revendications 16 à 18, **caractérisé en ce que** la contrainte au moins exercée est une contrainte due à l'humidité.

20. Lambris selon l'une des revendications 16 à 19, **caractérisé en ce que** la contrainte au moins exercée est une contrainte mécanique.

21. Lambris selon l'une des revendications 16 à 20, **caractérisé en ce que** la contrainte au moins exercée est une contrainte due à la lumière.

22. Lambris selon l'une de revendications 16 à 21, **caractérisé en ce que** l'élément de fixation au moins prévu est un lambris (3).

23. Lambris selon revendication 16 ou 22, **caractérisé en ce que** l'élément de fixation au moins prévu est une étiquette.

24. Lambris selon l'une des revendications 16 à 23, **caractérisé en ce que** l'élément de fixation au moins prévu est un matériau atténuant les bruits de pas.

25. Lambris selon l'une des revendications 16 à 24, **caractérisé en ce que** l'indicateur (5, 15) présente un recouvrement.

26. Lambris selon l'une des revendications 16 à 25, **caractérisé en ce qu'**est prévue au moins une échelle (13), qui explique l'apparence de l'indicateur (5, 15).

27. Lambris selon l'une des revendications 16 à 26, **caractérisé en ce que** l'indicateur (5, 15) est imprimé sur l'élément de fixation.
